# EUROPEAN PATENT SPECIFICATION

(11) **EP 2 915 546 B1**
(45) Date of publication and mention of the grant of the patent: **13.09.2017**
(21) Application number: 13850157.2
(22) Date of filing: 24.10.2013
(51) Int. Cl.: C02F 1/32

(54) **ULTRAVIOLET STERILIZER AND STERILIZATION METHOD**
UV-STERILISATOR UND UV-STERILISATIONSVERFAHREN
STÉRILISATEUR ULTRAVIOLET ET PROCÉDÉ DE STÉRILISATION

(30) Priority: 31.10.2012 JP 2012240250
(43) Date of publication of application: 09.09.2015
(73) Proprietor: Tokuyama Corporation, Shunan-shi, Yamaguchi-ken 745-8648 (JP)
(72) Inventor: MATSUI, Shingo, Shunan-shi Yamaguchi 745-8648 (JP)
(74) Representative: Markfort, Iris-Anne Lucie
(86) International application number: PCT/JP2013/006284
(87) International publication number: WO 2014/068913

(56) References cited:
- WO-A1-2010/058607
- WO-A1-2011/075694
- JP-A- H08 132 032
- JP-A- 2004 057 845
- JP-A- 2006 514 852
- US-A1- 2008 224 066
- US-A1- 2009 294 688

## Description

### Technical Field

The present invention relates to a novel ultraviolet disinfection apparatus and a disinfection method.

### Background Art

It is known that deep ultraviolet rays having a wavelength of 200 nm to 350 nm has a disinfection effect. An ultraviolet disinfection apparatus performing disinfection by irradiating such a deep ultraviolet rays is actually used. A typical light source used in the ultraviolet disinfection apparatus is a low pressure mercury lamp (so-called, disinfection lamp) radiating a light having a wavelength of 253.7 nm (mercury resonance line) generated by discharge of low pressure (about 0.1 Pa) mercury steam. The disinfection lamp is widely used for disinfection of water, powder and a container in a cooking room, a hospital or a pharmaceutical factory.

In recent years, an ultraviolet disinfection apparatus using modularized deep ultraviolet light-emitting diodes (hereinafter referred to as "deep ultraviolet LEDs") as a light source has been developed having a longer life and lower consumed power than the mercury lamp.

When fluid such as water is disinfected using an ultraviolet disinfection apparatus using such deep ultraviolet LEDs, the deep ultraviolet rays are generally irradiated while the fluid to be treated flows in terms of efficiency.

Specifically, as an apparatus where deep violet rays are irradiated while a light source is arranged in fluid to be disinfected, there is known an apparatus including an LED module arranging a plurality of deep ultraviolet LEDs on a surface inside of a treatment tank including an inlet part and an outlet part of an object to be treated and combining a rear surface of a first heat exchanger plate and a rear surface of a second exchanger plate, the respective exchange plates including a protection cover water-tightly covering the deep ultraviolet LEDs, in a state that a deep ultraviolet LED of the first heat exchanger plate is not overlapped back-to-back with a deep ultraviolet LED of the second heat exchanger plate (see Patent Document 1). As an apparatus where deep violet rays are irradiated while a light source is arranged outside of an object to be disinfected, there is known an apparatus including a light source arranged at a position sandwiched by a flow path made of bent quartz glass and cooled by the object to be disinfected flowing through the flow path (see Patent Document 2).

Patent Document 1: Japanese Patent Application Laid-open No. 2012-115715.
Patent Document 2: Japanese Patent Application Laid-open No. 2010-194414.
Further prior art: WO 2011/075694 A1 and US 2008/224066 A1.

### Summary of Invention

### Problem to be solved by the Invention

However, as the intensity of the deep ultraviolet rays radiated from the deep ultraviolet LEDs is significantly lower than the intensity of the deep ultraviolet rays radiated from the disinfection lamp, in order to perform sufficient disinfection, a number of deep ultraviolet LEDs should be arranged in line to prolong an irradiation time per unit amount of the object to be disinfected. There is a limitation to perform disinfection efficiently on a relatively large amount of the objects to be disinfected. For example, in the apparatuses described in Patent Documents 1 and 2, the deep ultraviolet LEDs are arranged planarly in the module used as the light source. In order to provide a required irradiation amount, each module itself has a large area. In addition, the number of the modules disposed within the treatment tank should be increased in the apparatus described in Patent Document 1 and a flow path length should be longer in Patent Document 2.

### Means for solving the Problem

The present invention solves the above-described problems by providing an ultraviolet disinfection apparatus with the features of claim 1.

In the apparatus of the present invention, it is preferable that a mechanism for generating a turbulence flow in the flow path is disposed.

The apparatus of the present invention includes a light condensing and ultraviolet light emitting unit including:
a light source including an ultraviolet light-emitting module where a plurality of "ultraviolet light-emitting devices having a disinfection action" is disposed on a side surface of a cylindrical or polygonal base such that a light axis of each ultraviolet light-emitting device passes through a center axis of the base and the ultraviolet rays are radially emitted to the center axis, and
a light condenser including a long elliptical reflective mirror or a parabolic reflective mirror,
the ultraviolet light-emitting module being disposed on a focal axis of the long elliptical reflective mirror or the parabolic reflective mirror to condense and emit the ultraviolet rays radially emitted from the ultraviolet light-emitting module, and
the object to be disinfected is irradiated with the ultraviolet rays emitted and condensed from the light condensing and ultraviolet light emitting unit.

More preferably, the apparatus of the present invention according to the above-described embodiments includes a plurality of the light condensing and ultraviolet light emitting units, the light condensing and ultraviolet light emitting units are arranged around the flow path, and the object to be disinfected is irradiated with the ultraviolet rays condensed multi-directionally.

In the disinfection apparatus of the present invention, the object is disinfected when an ultraviolet transmittance is 50% or less defined by a percentage of a ratio of transmitted ultraviolet intensity to irradiated ultraviolet intensity {(transmitted ultraviolet intensity/irradiated ultraviolet intensity) x 100(%)} when the object to be disinfected having a thickness of 1 cm is irradiated with ultraviolet rays having a disinfection action.

### Effect of the Invention

The ultraviolet disinfection apparatus of the present invention uses a light source including a plurality of "ultraviolet light-emitting devices (deep ultraviolet light-emitting devices) emitting ultraviolet rays (deep ultraviolet rays) having a disinfection action combined with a light condenser for condensing the ultraviolet rays emitted from each ultraviolet light-emitting device, thereby irradiating the object to be disinfected with deep ultraviolet rays having high intensity by the light condensing, which results in efficient disinfection. In the ultraviolet disinfection apparatus of the present invention, the object to be disinfected passes through the flow path and the light source and the light condenser are disposed externally to the flow path, thereby facilitating maintenance of the apparatus.

According to the ultraviolet disinfection apparatus of the present invention, the object to be disinfected is disinfected when the ultraviolet transmittance is 50% or less defined as described above, and the mechanism for generating the turbulence flow in the flow path is disposed, whereby the object to be disinfected flowing through the center part flows near a wall surface of the flow path, even when the ultraviolet rays irradiated from outside of the flow path is less likely to arrive at the object to be disinfected flowing through the center part of the flow path. Accordingly, if the length of the ultraviolet ray irradiation area is adequate, the object to be disinfected can be uniformly irradiated with ultraviolet rays, thereby performing sure disinfection.

With the ultraviolet disinfection apparatus of the present invention including the "light condensing and ultraviolet light emitting unit" including the light source combined with the light condenser, ultraviolet rays can be irradiated over an ultraviolet ray irradiation area having a rectangle shape and a high and uniform ultraviolet intensity, thereby down-sizing the apparatus. Furthermore, depending on an arrangement of the light condensing and ultraviolet light emitting unit, the irradiation area can be freely arranged. For example, when a plurality of the light condensing and ultraviolet light emitting units are arranged around the flow path and the object to be disinfected is irradiated with the ultraviolet rays condensed multi-directionally, the disinfection can be performed more efficiently. In addition, when the light condensing and ultraviolet light emitting units are arranged in line, an ultraviolet disinfection area can be lengthened.

### Brief Description of Drawings

[Fig. 1] A diagram showing transverse and longitudinal cross-sections of an ultraviolet light-emitting module suitably used as a light source in an ultraviolet disinfection apparatus according to one embodiment of the present invention.
[Fig. 2] A transverse cross-sectional view of a typical ultraviolet disinfection apparatus according to one embodiment of the present invention.
[Fig. 3] A side view of a typical ultraviolet disinfection apparatus according to one embodiment of the present invention.
[Fig. 4] A longitudinal cross-sectional view of a another ultraviolet disinfection apparatus according to one embodiment of the present invention.
[Fig. 5] A longitudinal cross-sectional view of a still another ultraviolet disinfection apparatus according to one embodiment of the present invention.

### Mode(s) for Carrying Out the Invention

An ultraviolet disinfection apparatus of the present invention is characterized by including:
a flow path surrounded by a material having a transmitting property to ultraviolet rays (deep ultraviolet rays) having a disinfection action through which a fluid object to be disinfected passes;
a light source disposed externally to the flow path for emitting ultraviolet rays having a disinfection action,
   the object to be disinfected passing through the flow path being disinfected by irradiating the ultraviolet rays emitted from the light source,
   the light source including a plurality of "ultraviolet light-emitting devices emitting ultraviolet rays having a disinfection action"; and
a light condenser for condensing the ultraviolet rays emitted from the ultraviolet light-emitting devices,
   the object to be disinfected being irradiated with the ultraviolet rays condensed by the light condenser.

Hereinafter, the ultraviolet disinfection apparatus of the present invention will be described referring to drawings taking a disinfection apparatus as an example, the disinfection apparatus including a "light condensing and ultraviolet light emitting unit" 130 including:
a light source including an ultraviolet light-emitting module where a plurality of deep ultraviolet light-emitting devices are disposed on a side surface of a cylindrical base such that a light axis of each deep ultraviolet light-emitting device passes through a center axis of the base and the deep ultraviolet rays are radially emitted to the center axis,
a light condenser including a long elliptical reflective mirror,
   the ultraviolet light-emitting module being disposed on a focal axis of the long elliptical reflective mirror to condense and emit the ultraviolet rays radially emitted from the ultraviolet light-emitting module.

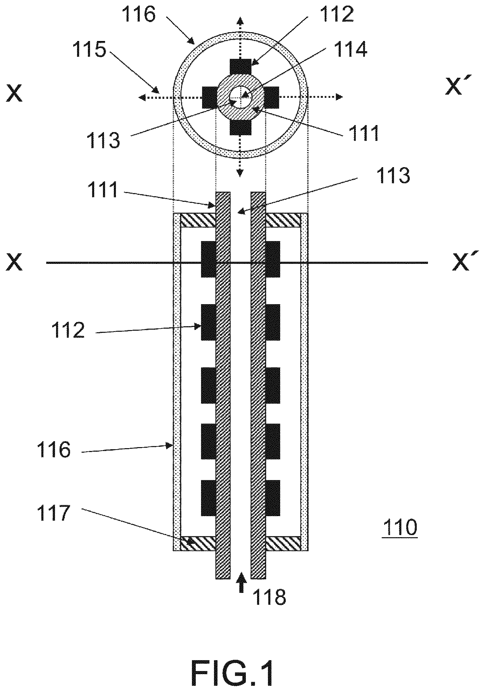

Fig. 1 shows transverse and longitudinal sections (cut at X-X' section) of an ultraviolet light-emitting module 110 used in the light condensing and ultraviolet emitting unit 130. As shown in the drawing, in the ultraviolet light emitting module 110, "a plurality of ultraviolet light-emitting devices emitting ultraviolet rays having a disinfection function" (also referred to as deep ultraviolet light-emitting diode) 112 is arranged in line on a surface of a cylindrical base 111, and a flow path for a cooling medium 113 is formed inside of the cylindrical base. The cylindrical base 111 to which the ultraviolet light-emitting devices 112 are mounted is covered with a cover 116 formed by an ultraviolet transmitting material such as quartz. The cover 116 is air-tightly or water-tightly mounted to the cylindrical base using a sealing member 117 such as an o-ring and is filled with an inert gas such as nitrogen or dry air gas. A desiccant (not shown) may be included in order to keep a water amount (humidity) to low within the cover. In this manner, the ultraviolet light-emitting device can have a high durability. The drawing shows an aspect where the cylindrical base, to which the ultraviolet light-emitting devices are mounted, uses a quartz tube as the cover.

As the deep ultraviolet light-emitting devices 112, LEDs (deep ultraviolet LEDs) emitting deep ultraviolet rays having a wavelength of 200 nm to 350 nm, preferably emitting deep ultraviolet rays having a wavelength of 240 nm to 290 nm are used. The deep ultraviolet LEDs are arranged in a state that the devices are mounted on a sub mount or in a state that they are housed in a package and that ultraviolet rays are preferably emitted in a constant direction. Although not shown, wiring for feeding electric power externally to the deep ultraviolet light-emitting devices, circuits for operating correctly the deep ultraviolet light-emitting devices and the like are formed on the sub mount or the package. Electrical power is fed to the wiring or the circuits via wiring formed on a surface of or inside of the cylindrical base 111.

The cylindrical base 111 functions not only as a support to fix and hold the deep ultraviolet light-emitting devices 112, but also as a heat sink. By passing a cooling medium 118 such as cooling water and cooling air through the flow path for a cooling medium 113 inside, a temperature increase caused by heat radiation from the ultraviolet light-emitting devices is prevented such that the devices are operated stably and device lives can be prolonged.

In the present invention, as a number of deep ultraviolet light-emitting devices are intimately mounted on the surface of the cylindrical base 111 as described later, it is especially important to remove efficiently heat generated at the ultraviolet light-emitting devices. Accordingly, the cylindrical base 111 is preferably mainly composed of a metal having a high thermal conductivity such as copper and aluminum and a ceramic. In addition, in order to increase a contact area of the cooling medium, it is preferable that an inner wall surface of the flow path for a cooling medium is subject to grooving. Further, when the cylindrical base 111 is composed of the metal material, it is preferable that an insulation layer is formed in order to insulate a copper wire or a circuit for feeding electrical power from an external power source to the ultraviolet light-emitting devices.

At a side surface of the cylindrical base 111, a plurality of the deep ultraviolet light-emitting devices 112 is arranged along a circumferential direction of the base such that extended lines of light axes 115 of the deep ultraviolet light-emitting devices 112 pass through the center axis 114 of the base 111 and light emitting surfaces are directed outwardly. As a result, ultraviolet rays emitted from the ultraviolet light-emitting devices emitted radially from the center axis 114. Note that the light axes 115 of the deep ultraviolet light-emitting devices 112 mean center axes of light beams emitted from the deep ultraviolet light-emitting devices and are almost synonymous to travel directions of the light beams. Also, note that "arranged such that the light axes 115 pass through the center axis 114 of the base 111" means that such a status is realized as much as possible and there is no problem that the light axis 115 is tilted slightly from the status.

Fig. 1 shows an example that four deep ultraviolet light-emitting devices are arranged in the circumferential direction of the base. But it is not limited thereto, and the arrangement can be changed depending on outer diameter of the cylindrical base 111, as appropriate. The number of the deep ultraviolet light-emitting devices arranged in the circumferential direction is generally 3 to 20, preferably 4 to 12. The higher the number of the ultraviolet light-emitting devices arranged in the circumferential direction is, the higher intensity of deep ultraviolet rays (photon flux density) emitted is. When a higher intensity of the ultraviolet rays is needed, the diameter of the cylindrical base 111 may be larger and the number of the deep ultraviolet light-emitting devices arranged in the circumferential direction may be increased exceeding the range.

The deep ultraviolet light-emitting devices 112 are preferably arranged so as to form lines in a longitudinal direction of the cylindrical base 111 as shown in the longitudinal cross-sectional view in Fig. 1. The longer an arrangement length in the longitudinal direction is, the longer the length of a deep ultraviolet ray irradiation area is. At this time, the deep ultraviolet light-emitting devices are preferably arranged intimately and regularly so as to provide a uniform intensity in the ultraviolet ray irradiation area.

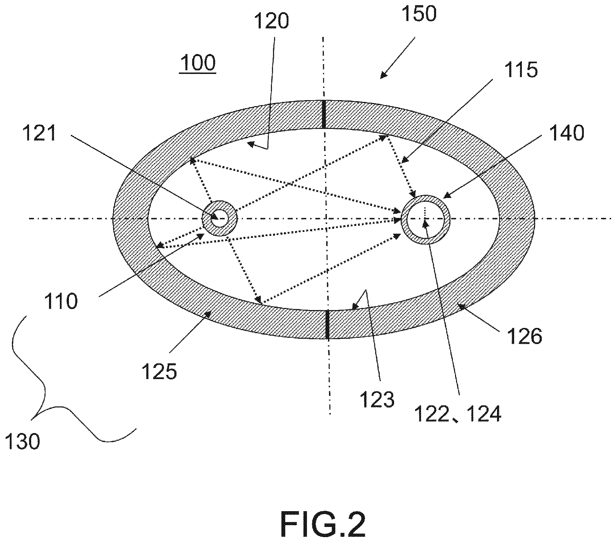

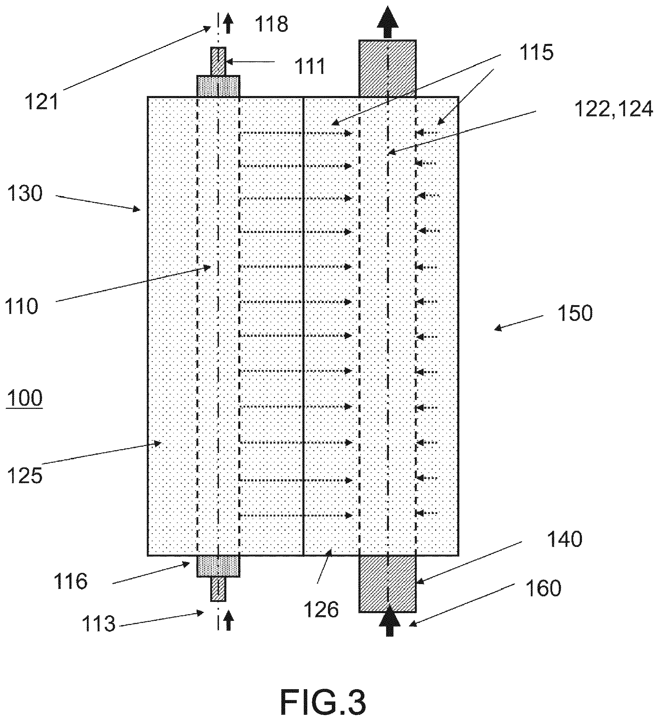

Fig. 2 and Fig. 3 show a transverse cross-sectional view and a longitudinal cross-sectional view of the ultraviolet disinfection apparatus 100 according to one embodiment of the present invention. The ultraviolet disinfection apparatus 100 has a main body 150 including an emitted-side housing 125 having inside an emitted-side reflective mirror 120 composed of a long elliptical reflective mirror and a light-condensed-side housing 126 having inside a light-condensed-side reflective mirror 123 composed of a long elliptical reflective mirror. Inside of the main body 150, the ultraviolet light-emitting module 110 and the flow path 140 are disposed. In the embodiment shown in the drawing, as the emitted-side reflective mirror 120 and the light-condensed-side reflective mirror 123 are the long elliptical reflective mirrors having substantially same shapes, in the main body 150, a light condensing axis 122 of the emitted-side reflective mirror 120 is coincide with a focal axis 124 of the light-condensed-side reflective mirror 123, and an inner space formed by binding the emitted-side housing 125 and the light-condensed-side housing 126 has a columnar shape having an ellipse cross-section where two axes of the a focal axis 121 of the emitted-side reflective mirror and a light condensing axis 122 of the emitted-side reflective mirror 124 are the focal axes.

In the main body 150, it is preferable that the emitted-side housing 125 and the light-condensed-side housing 126 can be mutually attached/detached or opened/closed using a hinge. At top and bottom openings on both sides of the main body 150 shown in Fig. 3, covers (not shown) for preventing ultraviolet rays from leaking to outside are disposed.

Surfaces of the emitted-side reflective mirror 120 and the light-condensed-side reflective mirror 123 are preferably composed of a material having a great reflectance to ultraviolet rays, e.g., a platinum group metal such as Ru, Rh, Pd, Os, Ir and Pt; Al, Ag, Ti; an alloy containing at least one of these metals; barium sulfate; or magnesium oxide. On the basis that the reflectance is especially high, it is especially preferable that they are formed of Al, a platinum group metal, an alloy containing the platinum group metal, barium sulfate or magnesium oxide. When they are composed of the metal material, in order to prevent surfaces from oxidizing or scratching and to prevent the reflectance from decreasing, it is preferable that the surface are coated with an ultraviolet transmitting material such as quartz and an ultraviolet transmitting dielectric.

In the ultraviolet light-emitting apparatus 100, the ultraviolet light-emitting module 110 is disposed such that the center axis 114 coincides with the focal axis 121 of the emitted-side reflective mirror. The light condensing and ultraviolet light emitting unit 130 is composed of the emitted-side housing 125 that is the light condenser including the emitted-side reflective mirror 120, and the ultraviolet light emitting module 110. In addition, a flow path 140 is arranged such that the center axis coincides with the light condensing axis 122 of the emitted-side reflective mirror (the focal axis 124 of the light-condensed-side reflective mirror). As the ultraviolet light emitting module 110 and the flow path 140 are disposed at the position, the deep ultraviolet rays emitted radially from the ultraviolet light emitting module 110 are reflected at the emitted-side reflective mirror and the light-condensed-side reflective mirror and are condensed converging on the focal axis of the light-condensed-side reflective mirror. The flow path 140 is effectively irradiated with the deep ultraviolet rays condensed. Thus, in the ultraviolet light-emitting apparatus 100, in principal, all deep ultraviolet rays emitted radially from the ultraviolet light emitting module 110 can be condensed on the focal axis of the light-condensed-side reflective mirror, and ultraviolet rays emitted not to the flow path 140 (for example, emitted to the opposite direction or the horizontal direction) can be effectively used.

The flow path 140 is surrounded by a material having transmitting properties to ultraviolet rays having a disinfection action, through which a fluid object to be disinfected 160 passes. Accordingly, the object to be disinfected 160 is irradiated with the deep ultraviolet rays transmitted through a surrounding diaphragm such as a quartz tube wall and a sapphire tube wall and is disinfected. The object to be disinfected 160 is a non-limiting liquid, and may be a gas such as air, a liquid such as water and a slurry where a small amount of a minute solid is suspended. When the object to be disinfected 160 flows to the flow path 140, a pretreatment that the object to be disinfected 160 passes through a filter or an adsorption layer is preferably performed in advance, as appropriate.

In order to perform sure disinfection, it is preferable that a mechanism for generating a turbulence flow in the flow path is disposed at the flow path 140. The mechanism for generating a turbulence flow is not especially limited as long as the turbulence flow is generated. Examples include a turbulence grid disposed within the flow path or an inlet of the flow path, irregularity formed on an inner wall surface, turbulence generation chips filled within the flow channel and a turbulence generation apparatus for randomly turning a impeller. When the flow of the object to be disinfected forms a so-called "laminar flow", the object to be disinfected flowing through the center part of the flow path may not be sufficiently irradiated with the deep ultraviolet rays, depending on the type of the object to be disinfected, the intensity of the deep ultraviolet rays irradiated and the diameter of the flow path. By generating the turbulence, the object to be disinfected flowing through the center part of the flow path can flow near a wall surface of the flow path. Accordingly, if the length of the ultraviolet ray irradiation area is adequate, the object to be disinfected can be uniformly irradiated with ultraviolet rays, thereby performing sure disinfection. In view of the above, attachment of the turbulence generation mechanism is effective when the object to be disinfected having a low deep ultraviolet transmittance, e.g., the object to be disinfected having an ultraviolet transmittance of 50% or less, especially 40% or less, is disinfected. The ultraviolet transmittance is defined by a percentage of a ratio of transmitted ultraviolet intensity to irradiated ultraviolet intensity {(transmitted ultraviolet intensity/irradiated ultraviolet intensity) x 100(%)} when the object to be disinfected having a thickness of 1 cm is irradiated with ultraviolet rays having a disinfection action. By attaching the turbulence generation mechanism, sure disinfection can be performed, even when the flow path has a large diameter. Therefore, a flow rate of the object to be disinfected can be increased and a disinfection efficiency can be further increased. A relationship between the ultraviolet transmittance and the thickness of a liquid phase is described in "Tsuneo Harada, et al.,: Germicidal lamp, Toshiba review, Vol. 6, No. 5, p289 (1951)", for example. According to this, the transmittance of milk, refined sake, beer, glucose injection (20%) or synthetic sake is 40% or less.

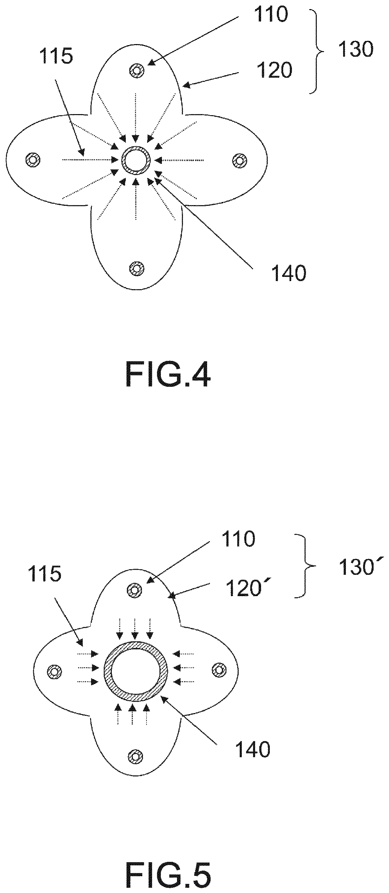

While the ultraviolet disinfection apparatus 100 shown in the drawings have been illustrated, it should be understood that the ultraviolet disinfection apparatus of the present invention is not limited thereto. For example, any light-condensed-side reflective mirror having a shape different from the long elliptical reflective mirror used as the emitted-side reflective mirror can be used as long as the ultraviolet rays are reflected to the emitted-side reflective mirror without leaking the ultraviolet rays to outside. As the emitted-side reflective mirror, a parabolic reflective mirror 120' can be used instead of the long elliptical reflective mirror. A light condenser including the parabolic reflective mirror 120' can be combined with the ultraviolet light-emitting module 110 to provide a light condensing and ultraviolet emitting unit 130'. A plurality of the light condensing and ultraviolet light emitting units 130 or light condensing and ultraviolet light emitting units 130' may be used. The light condensing and ultraviolet light emitting units may be arranged around the flow path 140 and the object to be disinfected may be irradiated with the ultraviolet rays condensed multi-directionally. Fig. 4 and Fig. 5 each shows an ultraviolet disinfection apparatus where four light condensing and ultraviolet light emitting units 130 or light condensing and ultraviolet light emitting units 130' are arranged around the flow path 140. By this arrangement, an amount of deep ultraviolet rays irradiated per unit of amount or per unit of time of the object to be disinfected can be increased.

### Description of Reference Numerals

100 ultraviolet disinfection apparatus
110 ultraviolet light-emitting module
111 cylindrical base
112 deep ultraviolet light-emitting device
113 flow path for cooling medium
114 center axis of cylindrical base
115 light axis of deep ultraviolet light-emitting device
116 cover
117 seal member
118 cooling medium
120 emitted-side reflective mirror composed of long elliptical reflective mirror
120' emitted-side reflective mirror composed of parabolic reflective mirror
121 focal axis of emitted-side reflective mirror
122 light condensing axis of emitted-side reflective mirror
123 light-condensed-side reflective mirror composed of long elliptical reflective mirror
124 focal axis of light-condensed-side reflective mirror
125 emitted-side housing
126 light-condensed-side housing
130 light condensing and ultraviolet light emitting unit (combination of 110 and 120)
130' light condensing and ultraviolet light emitting unit (combination of 110 and 120')
140 flow path for passing object to be disinfected
150 main body
160 object to be disinfected

## Claims

1. An ultraviolet disinfection apparatus (100) comprising:
a flow path (140) surrounded by a material having a transmitting property to deep ultraviolet rays having a disinfection action and having a wavelength of 200 nm to 350 nm through which a fluid object to be disinfected (160) passes;
a light condensing and deep ultraviolet light emitting unit (130) including:
a light source disposed externally to the flow path (140) for emitting the deep ultraviolet rays having a disinfection action,
the light source including an ultraviolet light-emitting module (110), comprising:
a base where a plurality of "ultraviolet light-emitting devices (112) for emitting deep ultraviolet rays" is disposed on a side surface of a cylindrical or polygonal columnar base (111) such that a light axis (115) of each ultraviolet light-emitting device (112) passes through a center axis (114) of the cylindrical or the polygonal columnar base (111) to emit the deep ultraviolet rays radially to the center axis (114); and
a cover (116) formed by a deep ultraviolet transmitting material,
the cover (116) covering the base where the ultraviolet light-emitting devices (112) are disposed, the cover being air-tightly mounted to the base, using a sealing member (117), where the inside of the cover is filled with an inert gas or dried air, and
a flow path (113) for a cooling medium being formed inside of the cylindrical or polygonal columnar base (111) to flow a cooling medium through the flow path (113) for a cooling medium, and a light condenser for condensing the ultraviolet rays emitted from the ultraviolet light-emitting devices, the light condenser including a long elliptical reflective mirror or a parabolic reflective mirror and condensing and emitting the deep ultraviolet rays radially emitted from the light source,
the ultraviolet light-emitting module (110) being disposed on a focal axis of the long elliptical reflective mirror or the parabolic reflective mirror (120, 120'), the object to be disinfected (160) being irradiated with the deep ultraviolet rays emitted by the light source and condensed by the light condensing and deep ultraviolet light emitting unit (130).

2. The ultraviolet disinfection apparatus (100) according to claim 1, **characterized in that**
a mechanism for generating a turbulence flow in the flow path (140) is disposed.

3. The ultraviolet disinfection apparatus (100) according to claim 1 or 2, **characterized in that**
a plurality of the light condensing and deep ultraviolet emitting units is included, the light condensing and deep ultraviolet emitting units (130, 130') are arranged around the flow path (140), and the object to be disinfected (160) is irradiated with the deep ultraviolet rays condensed multi-directionally.

4. The ultraviolet disinfection apparatus (100) according to any one of claims 1 to 3, **characterized in that**
the object is disinfected when an ultraviolet transmittance is 50% or less defined by a percentage of a ratio of transmitted deep ultraviolet intensity to irradiated deep ultraviolet intensity {(transmitted deep ultraviolet intensity/irradiated deep ultraviolet intensity) x 100(%)} when the object to be disinfected (160) having a thickness of 1 cm is irradiated with deep ultraviolet rays having a disinfection action and having a wavelength of 200 nm to 350 nm.

5. A disinfection method, **characterized by** using the ultraviolet disinfection apparatus (100) according to any one of claims 1 to 4, the object is disinfected when an ultraviolet transmittance is 50% or less defined by a percentage of a ratio of transmitted deep ultraviolet intensity to irradiated deep ultraviolet intensity {(transmitted deep ultraviolet intensity/irradiated deep ultraviolet intensity) x 100(%)} when the object to be disinfected (160) having a thickness of 1 cm is irradiated with deep ultraviolet rays having a disinfection action and having a wavelength of 200 nm to 350 nm.

## Patentansprüche

1. Ultraviolett-Desinfektionsanordnung (100) aufweisend:
einen Flussweg (140), der von einem Material umgeben ist, das eine Übertragungseigentschaft an tiefe ultraviolette Strahlen hat, die eine Desinfektionswirkung haben und eine Strahlenlänge von 200nm bis 350nm haben, durch welchen ein zu desinfizierendes Fluidobjekt (160) fließt;
eine Einheit zur Lichtkondensation und Emission von tiefem ultraviolettem Licht (130), umfassend:
eine Lichtquelle, die extern an dem Flussweg (140) angebracht ist, um die tiefen ultravioletten Strahlen, die eine Desinfektionswirkung haben, abzugeben, wobei die Lichtquelle ein Emissionsmodul zur Abgabe von ultraviolettem Licht (110) umfasst, aufweisend:
eine Basis, an der eine Vielzahl von Emissionsvorrichtungen zur Abgabe von ultraviolettem Licht (112) zum Abgeben von tiefen ultravioletten Strahlen, an einer Seitenfläche einer zylindrischen oder polygonalen säulenförmigen Basis (111) derart angebracht ist, dass eine Lichtachse (115) von jeder der Emissionsvorrichtungen zur Abgabe von ultraviolettem Licht (112) durch eine zentrale Achse (114) der zylindrischen oder polygonalen säulenförmigen Basis (111) läuft, um die tiefen ultravioletten Strahlen radial zu der zentralen Achse (114) abzugeben; und
eine Abdeckung (116) die aus einem Material geformt ist, das tiefe ultraviolette Strahlen überträgt, wobei die Abdeckung (116) die Basis dort abdeckt, wo die Emissionsvorrichtungen zur Abgabe von ultraviolettem Licht (112) angebracht sind, wobei die Abdeckung luftdicht an der Basis angebracht ist, unter Verwendung eines Dichtgliedes (117), dort, wo das Innere der Abdeckung mit einem Schutzgas oder getrockneter Luft gefüllt ist, und
einen Flussweg (113) für ein Kühlmedium, der im Inneren der zylindrischen oder polygonalen Basis (111) gebildet ist, damit ein Kühlmedium durch den Flussweg (113) für ein Kühlmedium fließen kann, und
einen Lichtkondensator zum Kondensieren von ultravioletten Strahlen, die von den Emissionsvorrichtungen zur Abgabe von ultraviolettem Licht abgegeben werden, wobei der Lichtkondensator einen langen elliptischen Reflektionsspiegel oder einen parabolischen Reflektionsspiegel umfasst und die tiefen ultravioletten Strahlen, die radial von der Lichtquelle abgegeben werden, kondensiert und abgibt,
wobei das Emissionsmodul zur Abgabe von ultraviolettem Licht (110) auf einer fokalen Achse des langen elliptischen Reflektionsspiegel oder des parabolischen Reflektionsspiegel (120, 120') angeordnet ist,
wobei das zu desinfizierende Objekt (160) mit den tiefen ultravioletten Strahlen bestrahlt wird, die von der Lichtquelle abgegeben werden und die von der Einheit zur Lichtkondensation und Emission von tiefem ultraviolettem Licht (130) kondensiert werden.

2. Ultraviolett-Desinfektionsanordnung (100) nach Anspruch 1,
**dadurch gekennzeichnet, dass** ein Mechanismus zum Erzeugen eines turbulenten Stroms in dem Flussweg (140) angeordnet ist.

3. Ultraviolett-Desinfektionsanordnung (100) nach Anspruch 1 oder 2,
**dadurch gekennzeichnet, dass** eine Vielzahl der Einheiten zur Lichtkondensation und Emission von tiefem ultraviolettem Licht (130, 130') um den Flussweg (140) herum angeordnet sind, und dass das zu desinfizierende Objekt (160) mit den tiefen ultravioletten Strahlen bestrahlt wird, die multidirektional kondensiert sind.

4. Ultraviolett-Desinfektionsanordnung (100) nach einem der Ansprüche 1 bis 3,
**dadurch gekennzeichnet, dass** das Objekt desinfiziert wird, wenn eine Durchlässigkeit für ultraviolette Strahlen bei 50% oder weniger definiert durch einen Prozentsatz eines Verhältnisses von durchgelassener tiefer ultravioletter Intensität zu einer bestrahlten tiefen ultravioletten Intensität {*(transmitted deep ultraviolet intensity* / *irradiated deep ultraviolet intensity) x 100*%} liegt, wenn das zu desinfizierende Objekt (160) mit einer Dicke von 1 cm mit den tiefen ultravioletten Strahlen bestrahlt wird, die eine Desinfektionswirkung haben und eine Strahlenlänge von 200nm bis 350nm haben.

5. Verfahren zum Desinfizieren, **gekennzeichnet durch** die Verwendung einer Ultraviolett-Desinfektionsanordnung (100) nach einem der Ansprüche 1 bis 4,
wobei das Objekt desinfiziert wird, wenn eine Durchlässigkeit für ultraviolette Strahlen bei 50% oder weniger definiert durch einen Prozentsatz eines Verhältnisses von durchgelassener tiefer ultravioletter Intensität zu einer bestrahlten tiefen ultravioletten Intensität {*(transmitted deep ultraviolet intensity* / *irradiated deep ultraviolet intensity) x 100*%*}* liegt, wenn das zu desinfizierende Objekt (160) mit einer Dicke von 1 cm mit den tiefen ultravioletten Strahlen bestrahlt wird, die eine Desinfektionswirkung haben und eine Strahlenlänge von 200nm bis 350nm haben.

## Revendications

1. Appareil de désinfection (100) aux ultraviolets, comprenant:
une trajectoire de flux (140) entourée d'un matériau ayant des propriétés de transmission de rayons ultraviolets profonds ayant une action désinfectante et ayant une longueur d'onde comprise entre 200 nm et 350 nm à travers lequel passe un objet fluide à désinfecter (160);
une unité (130) de condensation de la lumière et d'émission de lumière ultraviolette profonde comprenant :
une source de lumière disposée à l'extérieur de la trajectoire de flux (140) pour émettre les rayons ultraviolets profonds ayant une action désinfectante,
la source de lumière incluant un module (110) d'émission de rayons ultraviolets, comprenant :
une base où une pluralité de dispositifs émetteurs de rayons ultraviolets profonds (112) est disposée sur une surface latérale d'une base cylindrique ou sous forme de colonne polygonale (111), de telle manière qu'un axe de lumière (115) de chaque dispositif émetteur de rayons ultraviolets (112) passe par un axe central (114) de la base cylindrique ou sous forme de colonne polygonale (111) pour émettre les rayons ultraviolets profonds radialement vers l'axe central (114) ; et
un couvercle (116) formé avec un matériau transmettant les rayons ultraviolets profonds,
le couvercle (116) recouvrant la base où sont disposés les dispositifs émettant les rayons ultraviolets profonds (112), le couvercle étant monté de façon étanche à l'air sur la base, au moyen d'un organe de scellement (117), dans lequel l'intérieur du couvercle est rempli d'un gaz inerte ou d'air séché, et
un chemin de flux (113) pour un moyen de refroidissement étant ménagé à l'intérieur de la base cylindrique ou sous forme de colonne polygonale (111), pour permettre la circulation d'un moyen de refroidissement à travers le chemin de flux (113), et
un condenseur de lumière pour condenser les rayons ultraviolets émis par les dispositifs émetteurs de rayons ultraviolets, le condenseur de lumière incluant un long miroir elliptique réflecteur ou un miroir réflecteur parabolique qui condense et émet les rayons ultraviolets profonds émis par la source de lumière,
le module (110) émetteur de rayons ultraviolets étant disposé sur un axe focal du long miroir elliptique réflecteur ou du miroir réflecteur parabolique (120, 120'),
l'objet à désinfecter (160) étant irradié par les rayons ultraviolets profonds émis par la source de lumière, et condensés par l'unité (130) de condensation et d'émission de la lumière ultraviolette profonde.

2. Appareil de désinfection (100) aux ultraviolets, selon la revendication 1, **caractérisé en ce qu'**un mécanisme pour générer un flux turbulent est disposé dans la trajectoire de flux (140).

3. Appareil de désinfection (100) aux ultraviolets, selon la revendication 1 ou 2, **caractérisé en ce que**
une pluralité d'unités de condensation de la lumière et d'émission de rayonnement ultraviolets profonds est incluse, les unités (130, 130') de condensation de la lumière et d'émission de rayonnement ultraviolets profonds étant disposées autour de la trajectoire de flux (140), et l'objet à désinfecter (160) est irradié par les rayons ultraviolets profonds multidirectionnels.

4. Appareil de désinfection (100) aux ultraviolets, selon l'une quelconque des revendications 1 à 3, **caractérisé en ce que**
l'objet est désinfecté lorsqu'une transmittance ultraviolette est de 50% ou inférieure, définie par un pourcentage du ratio d'intensité d'ultraviolets profonds transmis, par rapport à l'intensité des rayons ultraviolets profonds irradiés {(intensité d'ultraviolets profonds transmis/intensité des rayons ultraviolets profonds irradiés) x 100%} lorsque l'objet à désinfecter (160), ayant une épaisseur de 1 cm est irradié avec des rayons ultraviolets profonds ayant une action désinfectante et une longueur d'onde de 200 nm à 350 nm.

5. Procédé de désinfection, **caractérisé par** l'utilisation d'un appareil de désinfection (100) aux ultraviolets, selon l'une quelconque des revendications 1 à 4, l'objet étant désinfecté lorsqu'une transmittance ultraviolette est de 50% ou inférieure, définie par un pourcentage du ration d'intensité d'ultraviolets profonds transmis, par rapport à l'intensité des rayons ultraviolets profonds irradiés {(intensité d'ultraviolets profonds transmis/intensité des rayons ultraviolets profonds irradiés) x 100%} lorsque l'objet à désinfecter (160), ayant une épaisseur de 1 cm est irradié avec des rayons ultraviolets profonds ayant une action désinfectante et une longueur d'onde de 200 nm à 350 nm.
